# EUROPEAN PATENT SPECIFICATION

(11) **EP 0 812 582 B1**
(45) Date of publication and mention of the grant of the patent: **02.05.2002**
(21) Application number: 97500104.1
(22) Date of filing: 09.06.1997
(51) Int. Cl.: A61F 2/38

(54) **Knee substitution prosthesis**
Knieprothese
Prothèse de genou

(30) Priority: 13.06.1996 ES 9601327
(43) Date of publication of application: 17.12.1997
(73) Proprietor: INDUSTRIAS QUIRURGICAS DE LEVANTE, S.A., 46988 Paterna - Valencia (ES)
(72) Inventor: Cogollos Munoz, Bernardo, 46988 Paterna Valencia (ES); Carreres Sancho, Benjamin, 46988 Paterna Valencia (ES); De Gracia Castillo de Olivares, Javier, 46988 Paterna Valencia (ES)
(74) Representative: Koepe, Gerd L., Dipl.-Chem.

(56) References cited:
- EP-A- 0 724 868
- EP-A- 0 729 732
- US-A- 4 714 475
- US-A- 5 314 481
- US-A- 5 370 701

## Description

### Introduction

Knee substitution prosthesis consisting of a new model of modular prosthetic knee formed by several components which, once assembled, configurate a hinged knee articulation. For such prupose, a metal femoral component, a metal tibial component and several elements (bolt, cylinder, polyethylene insert, etc.) for the attachment therebetween are provided, giving the degrees of freedom necessary for the prosthesis to ensure a stable, functional and low-friction articulation.

It is applicable in surgery in case of tumors affecting the distal third of a femur and/or the proximal third of tibia.

### BACKGROUND OF THE INVENTION

The knee substitution prosthesis of the present invention constitutes an improvement with respect to EP 0 729 732-A of Instituto Quirurgico de Levante, which provides a modular system for, as an example, a complete substitution of a femur (upper hip joint and lower knee joint) using the same connecting elements.

Patent US A 4 714 475 reveals a plate for fastening an artificial tendon into a cavity made in the tibial component to which said tendon is fastened by means of a single bolt, also used as a support means to stretch the artificial tendon, having the risk that when fastening said bolt the plate could follow a rotating movement. In that respect, the present invention reveals substantial advantages and improvements as compared to US A 4 714 475

Patent US A 5 314 481 reveals a knee joint comprising a considerable number of elements. The design of said joint is such that the rotation is effected at the lower tibial portion, below the joint The loads are transmitted through a pin or bolt, increasing the risk of destroying the prosthesis because of breaks, wearouts, friction, etc Said prosthesis is intended to be inserted into the tibia, and not to replace the same. Patent EP 97 500 104 of Industrias Quirurgicas de Levante refers to a rotation element located at the upper portion of the knee joint, wherein the connection to the femoral element through condylar surfaces is made with a small number of parts In the EP 97 500 104 the rotation movement is restricted by the bushing orifice and walls of tibial component. The loads are distributed over two large surfaces, the tibial insert upper surface and the condylar surface, in order to prevent overloads.

Patent EP 0 724 867 refers to a revision prosthesis to be inserted into the bone when a primary knee prosthesis has failed, whereas the present invention refers to an osseous substitution prosthesis involving a bone resection, the amplitude of which depends on the osseous tumor size

Patent US A 5 370 701 (D1) provides a flexo-extension movement requiring a considerable number of intermediate components (H) as shown on the drawing, which are subject to the loads transmitted by the femoral component, whereas in the present invention the loads are transmitted over two large surfaces such as the condylar surface and the tibial surface to prevent overloads, wearouts, breakages of intermediate parts, etc.

### DESCRIPTION

The present descrition relates to a new model of prosthetic knee to be used either in surgery of femoral or tibial tumors or in cases of revision, based on two metal (one femoral and one tibial) components, and several elements for the linking and hinging between both of them, giving three degrees of freedom and a stable articulation to the implant.

The presence of osseous tumors at femur or tibia requires levels of osseous resection much higher than conventional knee arthroplasty surgery. For this reason, the anchoring systems as well as the intrinsical stability of a primary prosthesis become insufficient.

In order to overcome this problem, prostheses for tumors have appeared on the market, wherein an articulation hinge between femur and tibia is typically provided to balance the lack of stability caused by the complete loss of the lateral and transverse ligaments. The articulation hinge constitutes a kinematic pair with only one degree of freedom (flexion-extension) so that rotational and abduction-adduction movements (occuring in a healthy knee) are hindered. Load transmission in prostheses of this kind is effected through the articulation hinge bolt, which often results in the breaking of the bolt.

In other models, rotation is effected either through a vertical pivot on which the femoral component rotates, or by the presence of a spherical ball-and-socket articulation vertically jutting out from the tibial plate and lodged within a spherical cavity between the condyles of the femoral component. In said two cases, it has not to do with kees for tumor surgery as such, but the mechanical solution is valid and constitutes a background. In all these rotational hinged knees there is no mobility between the tibial insert and the tibial metallic plate. The rotation occurs between prosthetic condyles and the upper portion of tibial insert, thereby giving rise to losses of congruence between the surfaces thereof and consequently to low contact areas, high tension and consequent wearout.

Rotation-less hinged prostheses have the drawbacks as mentioned above:
- Absence of rotation. Less functionality of articulation and higher stress at the prosthesis-bone interface.
- Transmission of load through the bolt. It limits the mechanical life-time of the prosthesis.

In order to improve the yield of a prosthesis in such cases, the implant design object of the present invention brings forward important improvements to the knee reconstruction surgery in cases of tumor.

The objective of the invention deals with a modular prosthetic system formed of various components which, once assembled, configurate a hinged knee articulation. For this purpose, a metal femoral component intended for substituting the distal portion of femur, a metal tibial component to substitute the proximal third of tibia, and several elements (bolt, cylinder, plastic insert, etc.) for the attachment between both of them are provided, giving the degrees of freedom necessary for the prosthesis to ensure a stable, functional and low-friction articulation.

Therefore, the knee substitution prosthesis herein proposed is susceptible to be integrated into a modular system to which it is possible to add components of different length in order to make it suitable for the level of osteotomy in each case.

The kinematic pair formed allows a relative rotation movement between femur and tibia. The prosthesis thereby allows more natural movements and the stresses between prosthesis and bone are lowered to a minimum.

The load does not pass through the bolt but through the condylar surfaces of femoral component.

This reduces the risk of failure of the bolt and represents a better approach to the load transmission model occuring in a healthy knee.

The friction pairs between condyles and tibial insert and between tibial insert and tibial plate are totally congruent. Cylinder-cylinder at the first stage and plane-plane at the second one. By this design, contact tension and thus wearout are minimized.

The design of the present invention will be better understood by means of the following drawings:

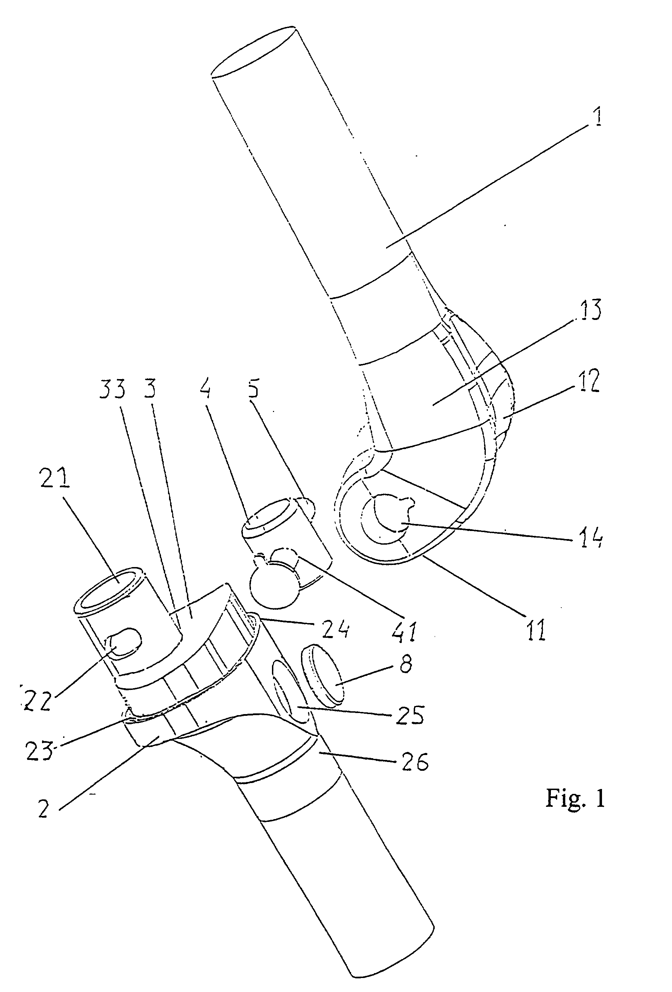

Figure 1.- Perspective view of the different pieces of a practical embodiment of the knee substitution prosthesis object of the present invention.

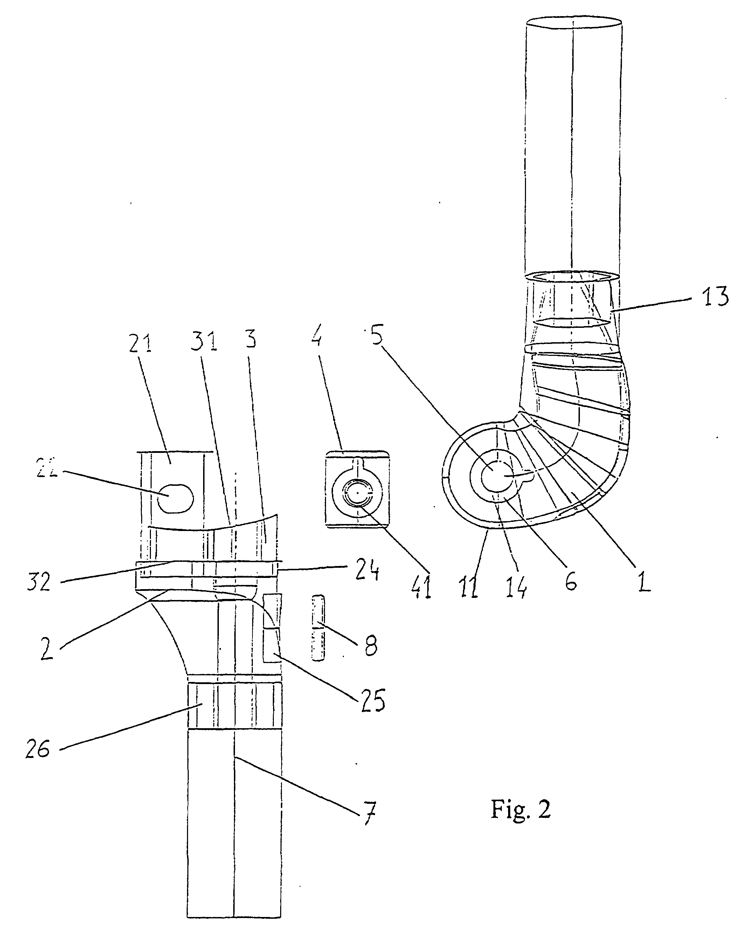

Figure 2.- Elevation view of different pieces forming the knee substitution prosthesis.

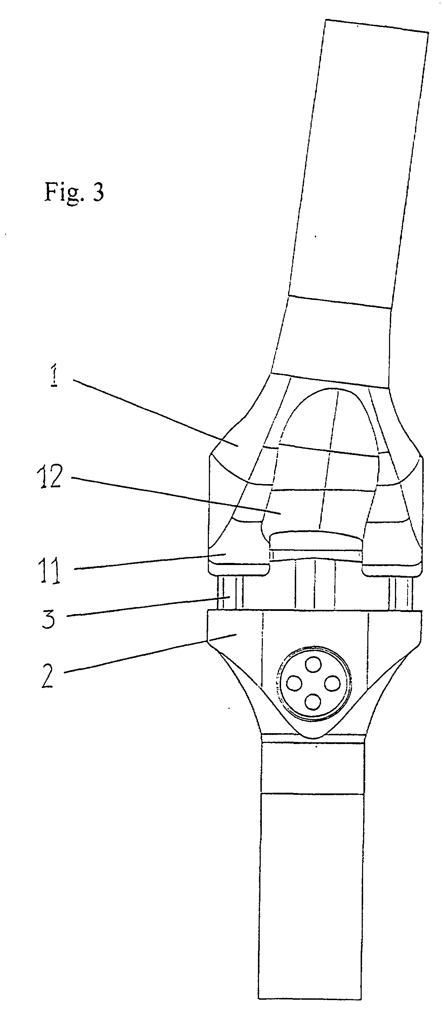

Figure 3.- Elevation view of the assembly of several pieces of the knee prosthesis.

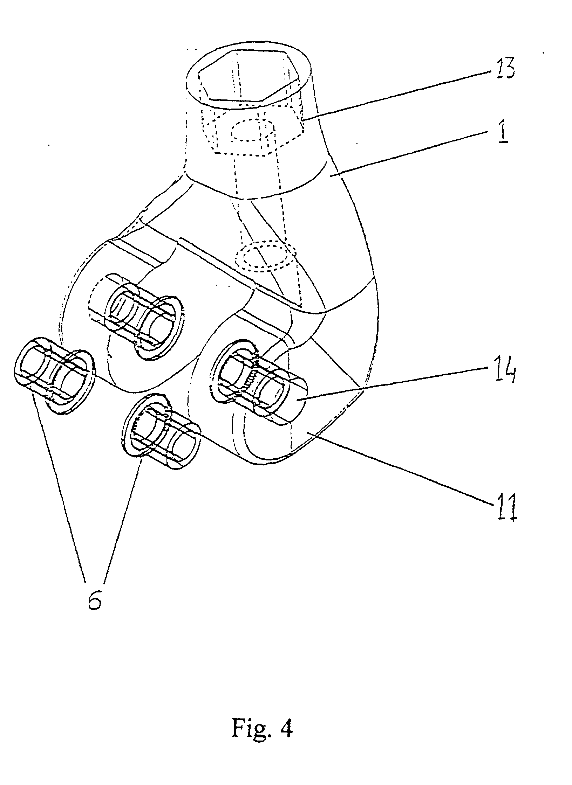

Figure 4.- Perspective view of a femoral component showing the detail of the bushings.

The knee substitution prosthesis, object of the present invention patent, is constituted by a femoral component, a tibial component and a series of attachment elements between both of them. The materials used for their construction is preferably, metal alloys common in the field of prosthetic surgery. More preferably, said materials are Chrome-Cobalt alloy (ASTM F75), or Titanium-Aluminium-Vanadium (Ti6 A14V ASTM F136). The tibial insert and the bushings are made of ultra-high molecular weight polyethylene UHMWPe.

The femoral component (1) consists of a solid metal piece with external shapes and dimensions similar to those of the distal end of the femur. At its lower portion, it has two cylindrical condylar surfaces (11) for the hinging with the tibial insert (3), and centrally, it has a concave portion or trochlea (12) on which the rotula slides. At the upper portion, the component has a hexagonal caving (13) where the hexagonal (male) end of an intramedullar rod is coupled, which is attached to the femoral component by a connecting pin or screw. Finally, the component has a cylindrical orifice (14) whose axis is concentric to the condylar surfaces and through which bushings (6) and bolt (5) are mounted.

The tibial portion (2) is also a metallic piece, shaped similarly to the proximal tibia. At its upper portion, a hollow cylindrical element (21) is extended with two bushing orifices horizontally elongated (22), that is, being the dimension in said horizontal direction larger than the orifice dimension in the vertical direction. This element constitutes the housing for a polyethylene cylinder (4) having a bushing orifice (41) through which bolt (5) runs. This bolt (5) freely rotates inside the hollow cylindrical end (21) of the tibial component. Thereby, causing the rotational and flexion-extension movements, respectively, of the knee.

The upper plane (23) of the tibial component constitutes the sliding surface on which the tibial insert (3) rotates. Consequently, this portion should be polished to reduce wearout. The upper plane has a projection (24) all along its perimeter acting as a stop in order to reduce the rotation movement. At its front side, the tibial component is provided with a caving (25) on which a polyethylene plate (8) is attached by screws or any other conventional method. The clamping of the patellar tendon may be carried out by the pressing (flattening) between caving (25) and plate (8).

Like the femoral component, the tibial component has, at its lower portion, an hexagonal caving for coupling an intramedullar rod fastening the tibial prosthesis. Said rod is attached to the tibial component by a connecting pin or screw (7) vertically running through the component.

The tibial insert (3) is an element made of ultra-high molecular weight polyethylene hinging at its upper portion (31) with the cylindrical condylar surfaces (11) of the femoral component and, at its lower portion (32), with the upper plane (23) of the tibial component. At its rear portion, it has a vertical cylindrical recess (33) hinging with the hollow cylindrical end (21) of the tibial component, which acts as an axis for the rotation movement of the insert.

Cylinder (4), bolt (5) and bushings (6) constitute the anchoring elements between femoral and tibial components. The bushings (6) are two hollow cylindrical pieces interposed between the orifices (14) of the femoral component (1) and the bolt (5) in order to reduce friction. This bolt is preferably, a metal cylindrical pin on which the articulation pivots during the flexion-extension movement. This bolt (5) runs through a cylindrical piece (4) made of ultra-high molecular weight polyethylene whose axis is vertical and which gets into the hollow cylinder (21) of the tibial component. The cylinder (4) may vertically move within its housing in the tibial component (2) in order to ensure that the load is transferred from condyles (11) of the femoral component (1) to the tibial insert (3) by direct contact and not through the bolt (5).

Bolt (5) and bushings (6) provide stability to the femoral component (1) at the front-rear plane and they are the axis or hinge for flexion-extension movements. Bolt (5) is held in its place within the cylinder thanks to a mechanical stop at one of its ends, avoiding the movement thereof in the left-right (longitudinal) direction.

The fixing of the femoral component (1) to femur is carried out by an intramedullar rod coated with a, preferably metal, porous layer in order to facilitate osteal integration. The hinging of the tibial components (2) is effected through the bolt (5) which allows the flexion-extension movement of knee. The load is transmitted through the two cylindrical surfaces (11) (condyles) to an insert (3) made of polyethilene which is mounted on the tibial plate. The friction surfaces (condyles and femoral trochlea) should be polished in order to minimize wearout

From all the foregoing, a knee substitution prosthesis with three degrees of freedom, two rotation and one shifting, whose connecting surfaces have a high congruence, therefore favouring the distribution of stresses, so that the material suffers less and lasts longer, thereby being capable of supporting higher stresses.

## Claims

1. Knee substitution prosthesis, comprising
a femoral component (1) having at a distal end two condylar surfaces (11), a cylindrical orifice (14) extending through said femoral component and having an axis concentric to said condylar surfaces (11) and bushings (6) which fit in said orifice (14), a tibial component (2) having at a proximal end a tibial insert (3), wherein a hollow cylinder (21) projects from the tibial insert (3), and a cylinder (4) which fits inside the hollow cylinder (21) on the tibial insert (3) allowing rotational movement,
said knee substitution prosthesis further comprises
a bolt (5) which fits through said cylindrical orifice (14) **characterised in that**
said bolt (5) further fits through a corresponding horizontally elongated orifice (22) in said hollow cylinder (21) and a orifice (41) in said cylinder (4),
the cylinder (4) moves vertically within the hollow cylinder (21) on the tibial component (2) in order to ensure that the load is transferred from condyles (11) of the femoral component (1) to the tibial insert (3) by direct contact and not through the bolt (5),
said components, forming a restricted, hinged articulation with three degrees of freedom: two rotational degrees, one about a vertical axis of the tibial insert and the tibial component and one about the bolt axis and acting on the condylar surfaces, corresponding respectively to the rotation and flexion-extension movements, and one shifting degree corresponding to the vertical distention movement, the shifting making possible to transmit the loads through the condylar surfaces.

2. Knee substitution prosthesis according to claim 1, **characterized in that** the knee flexion movement occurs between two congruent condylar surfaces, said surfaces being the condylar surface (31) of the tibial insert (3) and the condylar surface (11) of the femoral component (1).

3. Knee substitution prosthesis according to claim 1 **characterized in that** the tibial component (2) comprises an upper plane (23) which constitutes the sliding surface on which the lower portion (32) of the tibial insert (3) rotates, the upper plane (23) further comprises a projection (24) all along its perimeter acting as a stop in order to reduce the rotation movement.

4. Knee substitution prosthesis according to claim 1, **characterized in that** both the femoral component (1) and the tibial component (2) are provided with hexagonal cavings (13, 26) arranged at their respective ends for the anchoring of intramedular stems for the fastening onto the bone.

5. Knee substitution prosthesis according to claim 1, **characterized in that** the tibial component (2) is provided at the corresponding anatomical or longitudinal plane of said component (2) with a caving (25) in which a plastic plate (8) is inserted and fastened in order to configurate a hollow space allowing for the passage of the patellar tendon.

## Patentansprüche

1. Knieersatz-Prothese, umfassend
- eine Femur-Komponente (1), die aufweist: an einem distalen Ende zwei Kondylus-Flächen (11), eine zylindrische Öffnung (14), die sich durch die Femur-Komponente erstreckt und eine Achse, die mit den Kondylus-Flächen (11) konzentrisch ist, und Laufbuchsen (6) aufweist, die in die Öffnung (14) hineinpassen, eine Tibia-Komponente (2), die an einem proximalen Ende einen Tibia-Einsatz (3) aufweist, wobei ein Hohlzylinder (21) über den Tibia-Einsatz (3) hervorsteht, und einen Zylinder (4), der in den Hohlzylinder (21) auf dem Tibia-Einsatz (3) hineinpaßt und eine Drehbewegung erlaubt;
- wobei die Knieersatz-Prothese weiter umfaßt:
- einen Bolzen (5), der durch die zylindrische Öffnung (14) paßt, **dadurch gekennzeichnet daß**
- der Bolzen (5) weiter durch eine entsprechende horizontal längliche Öffnung (22) in dem Hohlzylinder (21) und eine Öffnung (41) in dem Zylinder (4) paßt;
- sich der Zylinder (4) vertikal innerhalb des Hohlzylinders (21) auf der Tibia-Komponente (2) bewegt und dadurch sicherstellt, daß sich die Belastung von den Kondylen (11) der Femur-Komponente (1) durch direkten Kontakt und nicht über den Bolzen (5) auf den Tibia-Einsatz (3) überträgt,
- wobei die Komponenten ein beschränktes, drehbares Gelenk mit drei Freiheitsgraden bilden, nämlich mit zwei Rotations-Freiheitsgraden, einem um eine vertikale Achse des Tibia-Einsatzes und der Tibia-Komponente und einem um die BolzenAchse und auf die Kondylus-Flächen wirkend, die jeweils den Bewegungen der Rotation und Biegung-Dehnung entsprechen, und einem Verschiebungs-Freiheitsgrad, der der Bewegung der vertikalen Ausdehnung entspricht, wobei es die Verschiebung ermöglicht, die Belastungen über die Kondylus-Flächen zu übertragen.

2. Knieersatz-Prothese nach Anspruch 1, **dadurch gekennzeichnet, daß** die Bewegung der Biegung des Knies zwischen zwei kongruenten Kondylus-Flächen erfolgt, wobei diese Flächen die Kondylus-Fläche (31) des Tibia-Einsatzes (3) und die Kondylus-Fläche (11) der Femur-Komponente (1) sind.

3. Knieersatz-Prothese nach Anspruch 1, **dadurch gekennzeichnet, daß** die Tibia-Komponente (2) eine obere Ebene (23) umfaßt, die die Gleitfläche darstellt, auf der sich der untere Abschnitt (32) des Tibia-Einsatzes (3) dreht, wobei die obere Ebene (23) im gesamten Bereich ihres Umfangs weiter einen Vorsprung (24) umfaßt, der als Stop dafür dient, um die Rotationsbewegung zu verringern.

4. Knieersatz-Prothese nach Anspruch 1, **dadurch gekennzeichnet, daß** sowohl die Femur-Komponente (1) als auch die Tibia-Komponente (2) mit hexagonalen Hohlräumen (13, 26), die an ihren jeweiligen Enden angeordnet sind, zum Verankern von intramedullären Stümpfen zur Befestigung am Knochen versehen sind.

5. Knieersatz-Prothese nach Anspruch 1, **dadurch gekennzeichnet, daß** die Tibia-Komponente (2) an der entsprechenden anatomischen oder Längsebene der Komponente (2) mit einem Hohlraum (25) versehen ist, in dem eine Kunststoff-Platte (8) eingesetzt und befestigt ist, um einen Hohlraum zu konfigurieren, der den Durchgang der Patella-Sehne erlaubt.

## Revendications

1. Prothèse de substitution du genou, comprenant :
un composant fémoral (1) ayant, au niveau d'une extrémité distale, deux surfaces condyliennes (11), un orifice cylindrique (14) s'étendant à travers ledit composant fémoral et ayant un axe concentrique par rapport auxdites surfaces condyliennes (11) et des douilles (6) qui sont ajustées dans ledit orifice (14), un composant tibial (2) ayant, au niveau d'une extrémité proximale, une garniture tibiale (3) dans laquelle un cylindre creux (21) s'étend à partir de la garniture tibiale (3), et un cylindre (4) qui est ajusté à l'intérieur du cylindre creux (21) sur la garniture tibiale (3) permettant un mouvement en rotation,
ladite prothèse de substitution du genou comprenant en outre :
un boulon (5) qui est ajusté à travers ledit orifice cylindrique (14), **caractérisé en ce que**
ledit boulon (5) est en outre ajusté à travers un orifice allongé horizontalement correspondant (22) dans ledit cylindre creux (21) et un orifice (41) dans ledit cylindre (4),
le cylindre (4) se déplace verticalement à l'intérieur du cylindre creux (21) sur le composant tibial (2) afin de garantir que la charge est transférée des condyles (11) du composant fémoral (1) sur la garniture tibiale (3) par un contact direct et non pas par l'intermédiaire du boulon (5),
lesdits composants, formant une articulation restreinte à charnière, à trois degrés de liberté : deux degrés de rotation, l'un autour d'un axe vertical de la garniture tibiale et du composant tibial, et l'autre autour de l'axe du boulon et agissant sur les surfaces condyliennes, correspondant respectivement aux mouvements de rotation et de flexion-extension, et un degré de déplacement correspondant au mouvement de distension verticale, le déplacement permettant de transmettre les charges par l'intermédiaire des surfaces condyliennes.

2. Prothèse de substitution du genou selon la revendication 1, **caractérisée en ce que** le mouvement de flexion du genou se produit entre deux surfaces condyliennes congruentes, lesdites surfaces étant la surface condylienne (31) de la garniture tibiale (3) et la surface condylienne (11) du composant fémoral (1).

3. Prothèse de substitution du genou selon la revendication 1, **caractérisée en ce que** le composant tibial (2) comprend un plan supérieur (23) qui constitue la surface de coulissement sur laquelle la partie inférieure (32) de la garniture tibiale (3) effectue une rotation, le plan supérieur (23) comprenant en outre une saillie (24) sur toute la longueur de son périmètre, qui agit comme butée afin de réduire le mouvement de rotation.

4. Prothèse de substitution du genou selon la revendication 1, **caractérisée en ce que** le composant fémoral (1) et le composant tibial (2) sont tous deux munis de cavités hexagonales (13, 26) agencées au niveau de leurs extrémités respectives pour l'ancrage de tiges intramédullaires pour la fixation dans l'os.

5. Prothèse de substitution du genou selon la revendication 1, **caractérisée en ce que** le composant tibial (2) est muni, au niveau du plan longitudinal ou anatomique correspondant dudit composant (2), d'une cavité (25) dans laquelle une plaque de plastique (8) est insérée et fixée afin de configurer un espace creux permettant le passage du tendon rotulien.
